# EUROPÄISCHE PATENTSCHRIFT

(11) **EP 0 630 643 B1**
(45) Veröffentlichungstag und Bekanntmachung des Hinweises auf die Patenterteilung: **02.08.1995**
(21) Anmeldenummer: 94108744.7
(22) Anmeldetag: 08.06.1994
(51) Int. Cl.: A61K 7/13, A61K 7/135

(54) **Mittel zum Färben und Blondieren von menschlichen Haaren und Verfahren zu dessen Herstellung**
Composition for dyeing or bleaching of human hair and method of preparation
Composition pour teindre ou blanchir des cheveux humains et méthode de préparation

(30) Priorität: 25.06.1993 DE 4321130; 07.10.1993 DE 4334183
(43) Veröffentlichungstag der Anmeldung: 28.12.1994
(73) Patentinhaber: GOLDWELL GmbH, D-64297 Darmstadt (DE)
(72) Erfinder: Lorenz, Heribert, D-64401 Gross-Bieberau (DE); Hirschfeld, Peter, D-79115 Freiburg i. Breisgau (DE); Eberling, Walter, D-64560 Riedstadt (DE)

(56) Entgegenhaltungen:
- EP-A- 0 560 088
- EP-A- 0 583 767
- Week 9347, Derwent Publications Ltd., London, GB; AN 93-374513 & JP-A-5 279 233 (SANYO CHEM IND LTD) 26. Oktober 1993

## Beschreibung

Die vorliegende Erfindung betrifft ein pulverförmiges Mittel zum Färben und Blondieren von menschlichen Haaren mit verbesserten Gebrauchseigenschaften und ein Verfahren zu dessen Herstellung.

Herkömmliche Mittel zum Blondieren bzw. Bleichen von menschlichen Haaren bestehen aus mindestens einem festen Peroxid, insbesondere einem Persulfat, und einem pulverförmigen Trägermaterial. Dieses Pulver wird bei Gebrauch mit einer 6- bis 12-prozentigen Wasserstoffperoxid-Lösung angerührt und auf das Haar aufgebracht. Beispiele für derartige Zusammensetzungen finden sich in der einschlägigen Fachliteratur, beispielsweise bei K.Schrader, "Grundlagen und Rezepturen der Kosmetika", 2.Aufl. (1989, Hüthig Buchverlag), S. 815 bis 823.

Die Gebrauchseigenschaften dieser Blondierpulver sind jedoch bisher nicht befriedigend. Sie stauben nicht nur bei der Anwendung, sondern sind auch bei der Dosierung nicht exakt handhabbar, wodurch das erwünschte Bleichergebnis beeinträchtigt werden kann.

Gleiches gilt für die bekannten pulverförmigen Mittel zum direkten Färben menschlicher Haare auf Basis von Naturfarbstoffen und direktziehenden Farbstoffen, die vor ihrer Anwendung mit Wasser angerührt werden.

Es wurde nunmehr gefunden, daß ein pulverförmiges Mittel zum Blondieren und Färben von menschlichen Haaren, das die erwähnten Nachteile nicht aufweist, erhalten werden kann, wenn das mindestens eine bleichaktive, insbesondere eine feste Per-Verbindung, enthaltende Mittel 5 bis 30 Gew.-% mindestens eines Wachses mit einem Schmelzpunkt zwischen 50 °C und 130 °C enthält.

Das so zusammengesetzte, mit dem Wachs agglomerierte bzw. überzogene Pulver ist nicht nur völlig staubfrei, sondern auch gut rieselfähig, gestattet eine exakte Dosierung und laßt sich daher problemlos mit Wasser bzw. einer Wasserstoffperoxid-Lösung vor Anwendung auf dem Haar vermischen.

Die Erfindung betrifft ferner ein pulverförmiges Mittel zum Blondieren oder Färben von menschlichen Haaren, enthaltend mindestens eine feste, bleichend wirkende Verbindung oder mindestens einen festen, direktziehenden Haarfarbstoff und 5 bis 30, insbesondere 10 bis 25 Gew.-%, mindestens eines C₁₂-C₁₈-Fettsäuremono- und/oder -dialkanolamids, insbesondere Cocosfettsäuremonoalkanolamids.

Aus der DE-OS 20 23 922 sind bereits granulierte Haarbleichmittel bekannt, die aus Persalzen und wasserlöslichen Bindemitteln, insbesondere Polyvinylpyrrolidon, hergestellt worden sind. Diese Granulate vermögen jedoch die oben angesprochenen Probleme deshalb nicht zu lösen, weil sie einerseits relativ hohe Teilchengrößen im Millimeterbereich aufweisen und beim Anrühren mit wäßrigem Wasserstoffperoxid nur schwer in Lösung gehen. Darüber hinaus ist auch die Herstellung dieser Produkte relativ aufwendig, im Gegensatz zu den erfindungsgemäßen Blondier- bzw. Farbpulvern, die vorzugsweise durch Verschmelzen des Wachses mit dem die feste bleichende Verbindung oder den pulverförmigen direktziehenden Haarfarbstoff enthaltenden Gemisch bzw. Aufsprühen des Wachses oder Alkanolamids auf dasselbe hergestellt werden.

Gleiches gilt für die aus der DE-OS 40 26 235 bekannten Granulate, die praktisch den aus der vorgenannten DE-OS 20 23 922 bekannten Produkten entsprechen.

Zur Herstellung der erfindungsgemäßen Blondierpulver sind im Prinzip alle natürlichen und synthetischen Wachse und wachsartigen Substanzen, deren Schmelzpunkt im optimalen Bereich von 50 °C bis 130 °C, insbesondere 50 °C bis 100 °C, liegt, geeignet, sofern sie das Lösungs- bzw. Dispergiervermögen der Zusammensetzung mit der wäßrigen Wasserstoffperoxid-Lösung nicht beeinträchtigen. Geeignete C₁₂-C₁₈-Fettsäuremonoalkanolamide und -dialkanolamide für pulverförmige Mittel zum Färben oder Blondieren können einen Schmelzpunkt von 50-130°C aufweisen.

Geeignete Wachse sind insbesondere Polyethylenglykol-Wachse mit einem Molgewicht zwischen 600 und 12.000, insbesondere 1.500 bis 10.000, wobei auch Gemische aus Polyethylenglykol-Wachsen höheren und niederen Molekulargewichts eingesetzt werden können, vorausgesetzt, der Schmelzpunkt des Gemisches liegt im erfindungsgemäßen Bereich.

Ein Beispiel für ein solches Gemisch ist eine Kombination aus 10 bis 30, insbesondere 20 Gew.-%, eines Polyethylenglykols mit einem Molgewicht von 400 bis 800, insbesondere etwa 600, und 70 bis 90, insbesondere etwa 80, Gewichtsteilen eines Polyethylenglykol-Wachses mit einem Molekulargewicht von 9.000 bis 11.000, insbesondere etwa 10.000.

Weitere geeignete Wachse sind beispielsweise Ester aus C₁₆-C₃₂-Fettsäuren und C₁₂-C₃₂-Fettalkoholen wie Cetylpalmitat, Mineralwachse, Bienenwachs, Schellackwachs, (hydrierte) Triglyceride, Lanolin und dessen Derivate, Walrat, Paraffinwachse, Mikrowachse, Ozokerit, Ceresinwachs, Candelillawachs, Carnaubawachs, Montanwachs, Japanwachs, Zuckerrohrwachs, Korkwachs, Guaramawachs, Astrolatum, Petrolatum, hydrierte Jojobawachse, Montanesterwachse, Mischwachse mit einem Gehalt an Emulgatoren wie die Handelsprodukte "Tegin®", "Lanette®", "Cutina®", "Dehymuls®" und "Emulgade®", Fettalkohole mit Wachskonsistenz, Fettsäureester mehrwertiger Alkohole wie z. B. Glycerinbehenat, Glycerinpalmitat/stearat, Polyglykolglyceride, Silikonwachse, höhere Paraffine.

Der Anteil der Wachse in den erfindungsgemäßen Zusammensetzungen liegt zwischen 5 und 30 Gew.-%, insbesondere zwischen 8 und 25 Gew.-%, besonders bei 10 bis 20 Gew.-%, jeweils berechnet auf die Gesamtzusammensetzung des Mittels.

Besonders geeignete C₁₂-C₁₈-Fettsäurealkanolamide sind Cocosfettsäuremonoethanolamid, Laurinfettsäuremonoethanolamid, Myristinfettsäuremonoethanolamid, Laurinmyristinsäuremonoethanolamid und Ölsäuremonoethanolamid, die entsprechenden Diethanolamide sowie auch Isopropanolamide, z. B. Laurinsäureisopropanolamid.

Verbessert werden können die Gebrauchseigenschaften des erfindungsgemsßen Blondier- oder Haarfärbepulvers noch durch die Mitverwendung geringer Mengen von Tensiden.

Als solche sind insbesondere anionische und nichtionische Produkte wie die bekannten C₁₂-C₁₈-Fettalkoholpolyglykolether, beispielsweise mit 3 bis 15 Ethylenoxid-Einheiten pro Mol, sowie Alkylphenolpolyglykolether, beispielsweise Nonylphenolpolyglykolether mit 4 bis 10 Ethylenoxid-Einheiten pro Mol, Glycerinstearate, Polyoxyethylensorbitanfettsäureester wie Polyoxyethylensorbitanmonooleat, -laurat oder -stearat oder Glycerinmono- oder -distearat, langkettige Alkylsulfate wie Natriumlaurylsulfat und Alkylethersulfate geeignet.

Deren Anteil liegt vorzugsweise bei 0,1 bis 2,5 Gew.-%, insbesondere bei 0,25 bis 1 Gew.-%, berechnet auf die Gesamtzusammensetzung des erfindungsgemäßen Mittels.

Ein weiterer bevorzugter Bestandteil in den erfindungsgemäßen Mitteln sind wasserlösliche Polymere, insbesondere Polyvinylpyrrolidon, vorzugsweise in einer Menge von 0,1 bis 1 Gew.-%, bezogen auf die Gesamtzusammensetzung.

Das erfindungsgemäße Mittel zum Blondieren bzw. Bleichen oder Färben von menschlichen Haaren enthält die in solchen Mitteln bekannten und üblichen Bestandteile, es wird hierzu, zur Vermeidung von Wiederholungen, auf Schrader, l.c., verwiesen.

Geeignete Peroxide sind insbesondere Alkalipersulfate wie Kalium- und Ammoniumpersulfat, Magnesiumperoxid, Harnstoffperoxid, Melaminperoxid, sowie Gemische derselben.

Es können jedoch auch Zusammensetzungen verwendet werden, die kein oder nur geringe Mengen Persulfat und statt dessen andere aufhellende Bestandteile, beispielsweise Ammoniumsalze wie Ammoniumcarbonat, Ammoniumbicarbonat, Ammoniumcitrate, Ammoniumphosphate, Ammoniumchlorid, Ammoniumsulfat und/oder Ammoniumcarbamat, enthalten.

Für die Herstellung des agglomerierten Farbpulvers können die bekannten Naturfarbstoffe eingesetzt werden, beispielsweise Henna (rot, schwarz oder neutral), Alkannin bzw. Alkannawurzelpulver, Blauholzpulver, Krappwurzel- und Rhabarberwurzelpulver.

Andere geeignete Farbstoffe sind synthetische direktziehende Farbstoffe, beispielsweise die bekannten "Arianor"-Farbstoffe, die alleine oder im Gemisch mit den Naturfarbstoffen Verwendung finden können.

Entsprechende Zusammensetzungen sind Stand der Technik und in einschlägigen Veröffentlichungen, beispielsweise bei Schrader, l.c., S. 782 bis 806, beschrieben.

Die Herstellung des erfindungsgemäßen Blondier- bzw. Haarfärbemittels erfolgt gemaß einer bevorzugten Ausführungsform der Erfindung durch Aufsprühen der geschmolzenen Wachse und der C₁₂-C₁₈-Fettsäurealkanolamide auf die restlichen Pulverbestandteile. Dies geschieht vorzugsweise bei 50 bis 130, insbesondere 50 bis 100 °C, je nach dem Schmelzpunkt des eingesetzten Wachses bzw. Fettsäurealkanolamids.

Eine andere Möglichkeit der Zumischung besteht darin, die Pulverbestandteile mit dem teilchenförmigen Wachs zu verwirbeln und anschließend zu verschmelzen und das erhaltene Agglomerat nach dem Abkühlen gegebenenfalls zu vermahlen.

Eine weitere erfindungsgemäße Verfahrensweise geht davon aus, zunächst einen Teil des zum Einsatz gelangenden Wachses als höherschmelzendes Wachs, beispielsweise Polyethylenglykol-Wachs mit einem Molgewicht von 8.000 bis 12.000, vorzugsweise etwa 10.000, mit der zu agglomerierenden bzw. zu überziehenden, pulverförmigen, eine bleichend wirkende Komponente und ein Trägermaterial enthaltenden Ausgangszusammensetzung bei 50 bis etwa 100 °C zu vermischen und anschließend den Rest des Wachses, beispielsweise ein Polyethylenglykol-Wachs mit einem Molgewicht von etwa 600, als niedrigschmelzendes Wachs flüssig aufzusprühen und die erhaltene Mischung anschließend abzukühlen.

Die Teilchengrößen der erfindungsgemäßen Blondier- und Färbemittel liegen in der Regel unterhalb 500 »m (Mikron), vorzugsweise unterhalb 400 »m (Mikron), was eine ausgezeichnete Verarbeitbarkeit, d.h. Mischbarkeit mit der wäßrigen Wasserstoffperoxid-Lösung vor Applikation auf das menschliche Haar, gewährleistet.

Die Anwendung des Mittels geschieht in an sich bekannter und üblicher Weise:
Durch Vermischen des pulverförmigen Blondiermittels mit einer 6- bis 12-prozentigen Wasserstoffperoxid-Lösung, wobei etwa 1 Teil des Pulvers mit etwa 1,5 Teilen der Peroxid-Lösung, vorzugsweise 9%iger H₂O₂-Lösung, homogen vermischt und anschließend 20 bis 60 Minuten auf das Haar zur Einwirkung gebracht wird.

Handelt es sich erfindungsgemäß um ein Färbemittel, wird dieses mit Wasser homogen angerührt und auf dem Haar zur Einwirkung gebracht.

Im folgenden werden einige Beispiele zur Realisierung der Erfindung gegeben.

### Beispiel 1

| | |
|---|---|
| Siliciumdioxid (Diatomeenerde) | 3,20 (Gew.-%) |
| Siliciumdioxid (pyrogenes SiO₂) | 5,30 |
| Natriumcarboxymethylcellulose | 3,50 |
| Harnstoff | 2,00 |
| Natriumlauroylsarkosinat | 0,80 |
| Natriumstearat | 1,20 |
| Natriumcarbonat | 1,00 |
| Natriummetasilikat | 6,00 |
| Stärkepulver | 3,50 |
| Kaliumpersulfat | 54,50 |
| Magnesiumperoxid | 4,00 |
| Cocosmonoethanolamid | 15,00 |

Es wurde ein staubfreies wasserlösliches bzw. -dispergierbares Pulver erhalten, das mit einer bekannten 9%igen H₂O₂-Lösung im Gewichtsverhältnis 1 : 1,5 gut angerührt werden konnte.

99 % der Teilchen hatten einen Durchmesser von < 400 »m (Mikron). Die Herstellung des Pulvers erfolgte durch Aufheizen der obengenannten Mischung auf 70 °C bis 75 °C in einem Wirbelstromgenerator und anschließendes Abkühlen.

### Beispiel 2

Die Zusammensetzung nach Beispiel 1 wurde dahingehend verändert, daß zusätzlich, unter entsprechender Verringerung des Gehaltes an Cocosmonoethanolamid, 0,15 Gew.-% eines C₁₂-C₁₄-Fettalkoholethoxylats (= 6 EO-Einheiten/Mol) zugesetzt wurden.

Es wurde ein fließfähiges staubfreies Produkt erhalten, das mit 9%iger H₂O₂-Lösung ausgezeichnet anrührbar war.

### Beispiel 3

Die Zusammensetzung nach Beispiel 1 wurde dahingehend verändert, daß zusätzlich, unter entsprechender Verringerung des Gehaltes an Cocosmonoethanolamid, 0,50 Gew.-% Natriumlaurylsulfat zugesetzt wurden.

Das erhaltene fließ- bzw. rieselfähige Blondierpulver war staubfrei und mit 9%iger H₂O₂-Lösung ausgezeichnet anrührbar.

### Beispiel 4

Eine Zusammensetzung aus:

| | |
|---|---|
| 32,00 (g) | Ammoniumpersulfat |
| 25,00 | Kaliumpersulfat |
| 10,50 | Natriummetasilikat |
| 5,50 | Siliciumdioxid |
| 4,80 | Stärke |
| 3,80 | Magnesiumperoxid |
| 3,20 | Harnstoff |
| 3,50 | Natriumcarboxymethylcellulose |
| 2,90 | Natriumstearat |
| 1,00 | Natriumlauroylsarkosinat |
| 2,50 | Diatomeenerde |
| 1,50 | Komplexbildner (Na EDTA) |
| 0,30 | Parfum |

wurde mit 18,00 g feinteiligem Cocosmonoethanolamid im Wirbelstrom bei 70 °C bis 75 °C besprüht.

Nach 15 Minuten wurde auf 25 °C abgekühlt; das erhaltene Agglomerat war nichtstaubend und ausgezeichnet mit 9%iger H₂O₂-Lösung zu einem homogenen Haarblondiermittel anrührbar.

Mehr als 99% der nach Beispiel 4 hergestellten Teilchen wiesen einen Durchmesser zwischen 50 »m und 500 »m auf. Der Wassergehalt lag jeweils unter 1 %.

### Beispiel 5

Eine Mischung aus

| | |
|---|---|
| 65,0 Gew.-% (Gew.-Teilen) | Alkannawurzel |
| 8,0 | Hennaschwarz |
| 2,0 | Natriumalginat |
| 2,0 | Natriumcarboxymethylcellulose |
| 2,5 | Natriumcarbonat |
| 0,5 | Natriumlaurylsulfat |
| 0,4 | Polyvinylpyrrolidon |

wurde in eine Wirbelschicht-Topsprayanlage eingebracht und mit 12 Gewichtsteilen Cocosfettsäuremonoethanolamid bei etwa 75 °C besprüht.

Das erhaltene Produkt war absolut staubfrei, besaß ausgezeichnete Rieselfähigkeit, war nichtklebend und ließ sich mit Wasser zu einem gut auf das Haar aufzutragenden Färbemittel anrühren, wobei nach 30- bis 60-minütiger Einwirkzeit ein graphitgrauer Farbton erhalten wurde.

Jeweils weniger als 1 % der Teilchen wies einen Durchmesser unterhalb 50 »m und oberhalb 900 »m auf.

### Beispiel 6

Ein Gemisch aus:

| | |
|---|---|
| 67,0 (Gew.-Teilen) | Hennarot |
| 8,0 | Hennaschwarz |
| 2,0 | Basic Blue 99 |
| 1,5 | Natriumalginat |
| 1,5 | Natriumcarboxymethylcellulose |
| 0,4 | Polyvinylpyrrolidon |
| 1,0 | Natriumlaurylsulfat und |
| 14,0 | Cocosfettsäuremonoethanolamid |

wurde vermischt und in einem Wirbelstromgenerator auf 70 °C bis 75 °C aufgeheizt und anschließend abgekühlt.

Das erhaltene Agglomerat ließ sich mit Wasser im Verhältnis 1 : 4 gut anrühren und ergab nach 30- bis 60-minütiger Einwirkung auf dem Haar einen dunkelbraunen Farbton.

### Beispiel 7

| | |
|---|---|
| Siliciumdioxid (Diatomeenerde) | 3,20 (Gew.-%) |
| Siliciumdioxid (pyrogenes SiO₂) | 5,30 |
| Natriumcarboxymethylcellulose | 3,50 |
| Harnstoff | 2,00 |
| Natriumlauroylsarkosinat | 0,80 |
| Natriumstearat | 1,20 |
| Natriumcarbonat | 1,00 |
| Natriummetasilikat | 6,00 |
| Stärkepulver | 3,50 |
| Kaliumpersulfat | 54,50 |
| Magnesiumperoxid | 4,00 |
| Polyethylenglykol-Wachs (MG 10.000) (Schmelzpunkt: 63 °C) | 15,00 |

Es wurde ein staubfreies wasserlösliches bzw. -dispergierbares Pulver erhalten, das mit einer bekannten 9%igen H₂O₂-Lösung im Gewichtsverhältnis 1 : 1,5 gut angerührt werden konnte.

99% der Teilchen hatten einen Durchmesser von < 400 »m (Mikron).

Die Herstellung des Pulvers erfolgte durch Aufheizen der obengenannten Mischung auf 70 bis 75 °C in einem Wirbelstromgenerator und anschließendes Abkühlen.

### Beispiel 8

Die Zusammensetzung nach Beispiel 7 wurde dahingehend verändert, daß zusätzlich, unter entsprechender Verringerung des Gehaltes an Polyethylenglykol-Wachs, 0,15 Gew.-% eines C₁₂-C₁₄-Fettalkoholethoxylats (≈ 6 EO-Einheiten/Mol) zugesetzt wurden.

Es wurde ein fließfähiges staubfreies Produkt erhalten, das mit 9%iger H₂O₂-Lösung ausgezeichnet anrührbar war.

### Beispiel 9

Die Zusammensetzung nach Beispiel 7 wurde dahingehend verändert, daß zusätzlich, unter entsprechender Verringerung des Gehaltes an Polyethylenglykol-Wachs, 0,50 Gew.-% eines Nonylphenolethoxylats (≈ 4 EO-Einheiten/Mol) zugesetzt wurden.

Das erhaltene fließ- bzw. rieselfähige Blondierpulver war staubfrei und mit 9%iger H₂O₂-Lösung ausgezeichnet anrührbar.

### Beispiel 10

Eine Zusammensetzung aus

| | |
|---|---|
| 32,00 (g) | Ammoniumpersulfat |
| 25,00 | Kaliumpersulfat |
| 10,50 | Natriummetasilikat |
| 5,50 | Siliciumdioxid |
| 4,80 | Stärke |
| 3,80 | Magnesiumperoxid |
| 3,20 | Harnstoff |
| 3,50 | Natriumcarboxymethylcellulose |
| 2,90 | Natriumstearat |
| 1,00 | Natriumlauroylsarkosinat |
| 2,50 | Diatomeenerde |
| 1,50 | Komplexbildner (Na EDTA) |
| 0,30 | Parfum |

wurde mit 18,00 g feinteiligem Polyethylenglykol-Wachs (MG 6.000) im Wirbelstrom bei 60 bis 70 °C besprüht.

Nach 15 Minuten wurde auf 25 °C abgekühlt; das erhaltene Agglomerat war nichtstaubend und ausgezeichnet mit 9%iger H₂O₂-Lösung zu einem homogenen Haarblondiermittel anrührbar.

### Beispiel 11

Zu einem Gemisch aus

| | |
|---|---|
| 40,0 (g) | Magnesiumperoxid |
| 330,0 | Ammoniumpersulfat |
| 250,0 | Kaliumpersulfat |
| 100,0 | Natriummetasilikat |
| 85,0 | Kieselsäure |
| 3,0 | Parfumöl |
| 28,0 | Natriumcarboxymethylcellulose |
| 0,5 | Blauer Farbstoff |
| 30,0 | Natriumstearat |
| 10,0 | Natriumlauroylsarkosinat |
| 10,0 | Komplexbildner |
| 50,0 | Stärke |
| 9,0 | Natriumcarbonat |

wurden in einem ersten Schritt 140 g Polyethylenglykol-Wachs mit einem Molgewicht von etwa 10.000 zugemischt, auf etwa 65 bis 75 °C erhitzt und anschließend, während etwa 10 bis 15 Minuten, 15 g Polyethylenglykol-Wachs mit einem Molgewicht von etwa 600 aufgesprüht. Nach dem Abkühlen wurde ein Blondierpulver-Agglomerat erhalten, das nicht staubte und mit 9%iger H₂O₂-Lösung leicht anzurühren war.
Mehr als 99 % der nach den Beispielen 10 und 11 hergestellten Teilchen wiesen einen Durchmesser zwischen 50 »m und 900 »m auf.

Der Wassergehalt lag jeweils unter 2,5 %.

## Patentansprüche

1. Pulverförmiges Mittel zum Blondieren von menschlichen Haaren, enthaltend mindestens eine feste, bleichend wirkende Verbindung und mindestens ein pulverförmiges Trägermaterial, dadurch gekennzeichnet, daß es 5 bis 30 Gew.-%, berechnet auf die Gesamtzusammensetzung, mindestens eines Wachses mit einem Schmelzpunkt zwischen 50 °C und 130 °C enthält.

2. Mittel nach Anspruch 1, dadurch gekennzeichnet, daß es 10 bis 25 Gew.-% mindestens eines Wachses mit einem Schmelzpunkt zwischen 50 °C und 130 °C enthält.

3. Mittel nach einem der Ansprüche 1 oder 2, dadurch gekennzeichnet, daß es als Wachs ein Polyethylenglykol-Wachs mit einem Molgewicht zwischen 600 und 12.000 enthält.

4. Mittel nach einem der Ansprüche 1 oder 2, dadurch gekennzeichnet, daß es als Wachs ein Gemisch aus 70 bis 90 Gewichtsteilen Polyethylenglykol mit einem Molgewicht von 9.000 bis 11.000 und 10 bis 30 Gew.-% Polyethylenglykol mit einem Molgewicht von 400 bis 800 enthält.

5. Mittel nach einem der vorhergehenden Ansprüche, dadurch gekennzeichnet, daß es 0,1 bis 1 Gew.-% eines nichtionischen Tensids enthält.

6. Mittel nach Anspruch 5, dadurch gekennzeichnet, daß es als nichtionisches Tensid einen Fettalkoholpolyglykolether und/oder einen Alkylphenolpolyglykolether enthält.

7. Verfahren zur Herstellung eines pulverförmigen Mittels zum Blondieren von menschlichen Haaren nach einem der Ansprüche 1 bis 6, dadurch gekennzeichnet, daß auf ein mindestens eine feste, bleichend wirkende Verbindung enthaltendes Pulver mindestens ein geschmolzenes Wachs mit einem Schmelzpunkt zwischen 50 °C und 130 °C aufgesprüht wird.

8. Verfahren zur Herstellung eines pulverförmigen Mittels zum Blondieren von menschlichen Haaren nach einem der Ansprüche 1 bis 6, dadurch gekennzeichnet, däß ein mindestens eine bleichend wirkende Verbindung enthaltendes Pulver mit einem Wachs bzw. einer wachsartigen Substanz mit einem Schmelzpunkt zwischen 50 °C und 130 °C vermischt und anschließend bei einer den Schmelzpunkt des eingesetzten Wachses übersteigenden Temperatur verschmolzen und anschließend abgekühlt wird.

9. Pulverförmiges Mittel zum Färben oder Blondieren von menschlichen Haaren, enthaltend mindestens eine feste, bleichend wirkende Verbindung oder mindestens einen festen, direktziehenden Haarfarbstoff, dadurch gekennzeichnet, daß es 5 bis 30 Gew.-%, berechnet auf die Gesamtzusammensetzung, mindestens eines C₁₂-C₁₈-Fettsäuremonoalkanolamids und/oder -dialkanolamids mit einem Schmelzpunkt zwischen 50 und 130°C enthält.

10. Mittel nach Anspruch 9, dadurch gekennzeichnet, daß es als C₁₂-C₁₈-Fettsäuremonoalkanolamid Cocosfettsäuremonoalkanolamid enthält.

11. Mittel nach einem der Ansprüche 9 oder 10, dadurch gekennzeichnet, daß es zusätzlich 0,1 bis 5 Gew.-% eines Tensids enthält.

12. Mittel nach Anspruch 11, dadurch gekennzeicnnet, daß es 0,1 bis 2,5 Gew.-% eines nichtionischen Tensids enthält.

13. Mittel nach Anspruch 11, dadurch gekennzeichnet, daß es 0,1 bis 2,5 Gew.-% eines anionischen Tensids enthält.

14. Verfahren zur Herstellung eines pulverförmigen Mittels zum Färben oder Blondieren von menschlichen Haaren nach einem der Ansprüche 9 bis 13, dadurch gekennzeicnnet, daß auf ein mindestens eine feste, bleichend wirkende Verbindung oder einen direktziehenden Haarfarbstoff enthaltendes Pulver mindestens ein geschmolzenes C₁₂-C₁₈-Fettsäuremono- und/oder -dialkanolamid bei einer Temperatur zwischen 40 °C und 130 °C aufgesprüht wird.

15. Verfahren zur Herstellung eines pulverförmigen Mittels zum Färben oder Blondieren von menschlichen Haaren nach einem der Ansprüche 9 bis 13, dadurch gekennzeichnet, daß ein mindestens eine bleichend wirkende Verbindung oder einen direktziehenden Haarfarbstoff enthaltendes Pulver mit einem C₁₂-C₁₈-Fettsäuremono- und/oder -dialkanolamid mit einem Schmelzpunkt zwischen 50 und 130°C vermischt und anschließend bei einer den Schmelzpunkt des eingesetzten C₁₂-C₁₈-Fettsäuremonoalkanolamids übersteigenden Temperatur verschmolzen, anschließend abgekühlt und agglomeriert wird.

## Claims

1. Pulverulent composition for bleaching of human hair, containing at least one solid active bleaching compound and at least one pulverulent carrier material, characterized in that it contains 5% to about 30% by weight, calculated to the total composition, of at least one wax compound having a flow point between 50°C and 130°C.

2. Composition according to claim 1, characterized in that it comprises 10% to 25% by weight of at least one wax compound having a flow point between 50°C and 130°C.

3. Composition according to one of the claims 1 or 2, characterized in that it contains as wax compound a polyethylene glycol wax having a molecular weight between 600 and 12,000.

4. Composition according to one of the claims 1 or 2, characterized in that it contains a wax mixture of 70 to 90 parts by weight of a polyethylene glycol having a molecular weight of 9,000 to 11,000, and 10 to 30 parts by weight of a polyethylene glycol having a molecular weight of 400 to 800.

5. Composition according to one of the preceding claims, characterized in that it additionally comprises about 0.1% to about 1% by wt. of a nonionic surfactant.

6. Composition according to claim 5, characterized in that it comprises a fatty alcohol polyglycol ether and (or) an alkylphenol polyglycol ether as the nonionic surfactant.

7. Process for the preparation of a pulverulent composition for bleaching of human hair according to one or more of the claims 1 to 6, characterized in that a wax compound having a flow point between 50°C and 130°C is sprayed onto a powder comprising at least one solid active bleaching compound, at a temperature between 50°C and 130°C.

8. Process for the preparation of a pulverulent composition for bleaching or dyeing of human hair according to one or more of the claims 1 to 6, characterized in that at least one active bleaching compound is mixed with a wax compound having a flow point between 50°C and 130°C, and melted thereafter at a temperature exceeding the flow point of the wax compound, whereafter the mixture is cooled down.

9. Pulverulent composition for dyeing and bleaching of human hair, containing at least one solid active bleaching compound or at least one solid direct dyestuff, characterized in that it contains 5% to 30% by wt., calculated to the total composition, of at least one C₁₂-C₁₈-fatty acid mono- and (or) dialkanolamide, having a flow point from 50°C to 130°C.

10. Composition according to claim 9, characterized in that it contains coconut fatty acid monoalkanolamide as a C₁₂-C₁₈-fatty acid monoalkanolamide.

11. Composition according to one of claims 9 or 10, characterized in that it additionally contains 0.1% to 5% by wt. of a surfactant.

12. Composition according to claim 11, characterized in that it contains 0.1% to 2.5% by wt. of a nonionic surfactant.

13. Composition according to claim 11, characterized in that it contains 0.1% to 2.5% by wt. of an anionic surfactant.

14. Process for the preparation of a pulverulent composition for dyeing or bleaching of human hair according to claims 9 to 13, characterized in that at least one melted C₁₂-C₁₈-fatty acid mono- and (or) dialkanolamide is sprayed onto a powder comprising at least one solid compound exerting bleaching activity upon application on human hair or a direct hair dye at a temperature between 40°C and 130°C.

15. Process for the preparation of a pulverulent composition for dyeing or bleaching of human hair according to one of the claims 9 to 13, characterized in that a powder containing at least one compound exerting bleaching activity upon application on human hair or a direct dye is mixed with a C₁₂-C₁₈-fatty acid mono- and (or) dialkanolamide having a flow point from 50°C to 130°C, and subsequently melted at a temperature exceeding the melting point of the C₁₂-C₁₈-fatty acid monoalkanolamide used, whereupon the mixture is cooled and agglomerized.

## Revendications

1. Composition en forme de poudre pour blondir les cheveux humains, contenant au moins un composé solide agissant comme décolorant et au moins un support en forme de poudre, caractérisé en ce qu'elle contient de 5 à 30 % en poids, par rapport à la composition totale, d'au moins une cire ayant un point de fusion entre 50 °C et 130 °C.

2. Composition selon la revendication 1, caractérisée en ce qu'elle contient de 10 à 25 % en poids d'au moins une cire ayant un point de fusion entre 50 °C et 130 °C.

3. Composition selon l'une des revendications 1 ou 2, caractérisée en ce qu'elle contient comme cire, une cire de polyéthylène glycol ayant un poids moléculaire entre 600 et 12 000.

4. Composition selon l'une des revendications 1 ou 2, caractérisée en ce qu'elle contient comme cire, un mélangé constitué par 70 à 90 parties en poids de polyéthylène glycol ayant un poids moléculaire entre 9 000 et 11 000, et 10 à 30% en poids de polyéthylène glycol ayant un poids moléculaire entre 400 et 800.

5. Composition selon l'une des revendications précédentes, caractérisée en ce qu'elle contient de 0,1 à 1 % en poids d'un agent tensio-actif non ionique.

6. Composition selon la revendication 5, caractérisée en ce qu'elle contient comme agent tensio-actif non ionique un polyglycoléther d'alcool gras et/ou un polyglycoléther d'alkylphénol.

7. Procédé pour la préparation d'une composition en forme de poudre pour blondir les cheveux humains selon l'une des revendications 1 à 6, caractérisé en ce que l'on pulvérise sur une poudre contenant au moins un composé solide agissant comme décolorant, au moins une cire fondue ayant un point de fusion entre 50 °C et 130 °C.

8. Procédé pour la préparation d'une composition en forme de poudre pour blondir les cheveux humains selon l'une des revendications 1 à 6, caractérisé en ce qu'une poudre contenant au moins un composé agissant comme décolorant est mélangée avec une cire ou une substance analogue à une cire ayant un point de fusion entre 50 °C et 130 °C, puis fondue à une température supérieure à la température de fusion de la cire et ensuite refroidie.

9. Composition en forme de poudre pour colorer ou blondir les cheveux humains, contenant au moins un composé solide agissant comme décolorant ou au moins un colorant solide pour cheveux agissant directement sur le cheveu, caractérisé en ce qu'elle contient de 5 à 30 % en poids, par rapport à la composition totale, d'au moins un monoalcanolamide et/ou un dialcanolamide d'acide gras en C₁₂-C₁₈, ayant un point de fusion entre 50 et 130°C.

10. Composition selon la revendication 9, caractérisée en ce qu'elle contient un monoalcanolamide de l'huile de coprah en tant que monoalcanolamide d'acide gras en C₁₂-C₁₈.

11. Composition selon l'une des revendications 9 ou 10, caractérisée en ce qu'elle contient en outre de 0,1 à 5 % en poids d'un agent tensio-actif.

12. Composition selon la revendication 11, caractérisée en ce qu'elle contient de 0,1 à 2,5 % en poids d'un agent tensio-actif non ionique.

13. Composition selon la revendication 11, caractérisée en ce qu'elle contient de 0,1 à 2,5 % en poids d'un agent tensio-actif anionique.

14. Procédé pour la préparation d'une composition en forme de poudre pour colorer ou blondir les cheveux humains selon l'une des revendications 9 à 13, caractérisé en ce que l'on pulvérise sur une poudre contenant au moins un composé solide agissant comme décolorant ou au moins un colorant pour cheveux agissant directement sur le cheveu, au moins un monoalcanolamide et/ou un dialcanolamide d'acide gras en C₁₂-C₁₈ fondu, à une température entre 40 °C et 130 °C.

15. Procédé pour la préparation d'une composition en forme de poudre pour colorer ou blondir les cheveux humains selon l'une des revendications 9 à 13, caractérisé en ce qu'une poudre contenant au moins un composé agissant comme décolorant ou au moins un colorant pour cheveux agissant directement sur le cheveu, est mélangée avec un monoalcanolamide et/ou un dialcanolamide d'acide gras en C₁₂-C₁₈ ayant un point de fusion entre 50 °C et 130 °C, ensuite fondue à un température supérieure à la température de fusion du monoalcanolamide d'acide gras en C₁₂-C₁₈ introduit, ensuite refroidie et agglomérée.
